# EUROPEAN PATENT APPLICATION

(11) **EP 2 420 842 A1**
(43) Date of publication of application: **22.02.2012**
(21) Application number: 10008628.9
(22) Date of filing: 18.08.2010
(51) Int. Cl.: G01N 33/68, C07K 14/71

(54) **In vivo imaging of calcified lesions**

(71) Applicant: Rheinisch-Westfälische Technische Hochschule Aachen (RWTH), 52062 Aachen (DE)
(72) Inventor: Schäfer, Cora, 65428 Rüsselsheim (DE)
(74) Representative: Lahrtz, Fritz

(57) **Abstract**

The present invention relates to a fetuin polypeptide for use in a method for diagnosis of calcification.

## Description

The present invention relates to a fetuin polypeptide for use in a method for diagnosis of calcification.

Calcification is a widespread and highly important process characterized by the deposition of calcium salts. *In vivo,* calcification plays an important role in the mineralization of bones and teeth, in particular in the formation, the growth and the regeneration of bones and teeth. Therefore, calcification is an indispensable process for most animals, including humans.

However, calcification may also occur in other tissues and organs and may, hereby, be pathological. For instance, calcification may cause soft tissues such as the skin, brown fat, lung, kidney, heart or a j oint to harden. Here; calcification may lead to a pathological tissue and potentially to a corresponding pathological condition including the clinical symptoms thereof. Several types of tumours such as certain types of brain tumours bear a distinctive local calcification (calcium spots). Alternatively, calcium spots of different sizes may occur in the brain without provoking clinical symptoms or indicating any pathological condition.

Furthermore, calcification may occur in the lumen of certain organs such as the kidney, in particular the renal pelvis, the ureter, the bladder, the gallbladder and the bile duct. Here, a kidney stone (nephrolith) and a gallstone, respectively, may occur.

Nevertheless, the most common pathological calcification is atherosclerosis. Mild forms of atherosclerosis mostly occur in elderly people without provoking any clinical symptoms. However, cardiovascular calcification may increase the risk of numerous diseases such as hypertonia, myocardial infarction and cerebral apoplexy (stroke). Highly pronounced forms of calcification may further lead to the necessity of amputation of an extremity such as a leg.

The process of *in vivo* calcification is not fully understood so far. However, different chemical factors are known that may play an important role in calcium metabolism and homeostasis. Such chemical factors may be *inter alia* vitamin D, oxalic acid, cortisol and calcium-binding proteins.

One of the most important calcium-binding proteins is fetuin-A. Fetuin-A is a liver-derived serum protein involved in mineral homeostasis. Fetuin-A deficient mice show widespread soft tissue calcifications supporting the notion that fetuin-A is a mineral chaperone mediating the stabilization of protein-mineral complexes and their clearing in the body (Heiss A et al., J Biol Chem 2003;278:13333-13341; Jahnen-Dechent W et al., J Mol Med. 2008;86(4):379-389). Fetuin-A deficient mice develop pathological calcification in soft tissues (Schäfer C et al., J Clin Invest. 2003;112(3):357-366; Merx M et al., J Am Soc Nephrol. 2005;16(11):3357-3364). Fetuin-A deficient mice against the genetic background C57BL/6 develop little spontaneous calcification, but readily calcify in soft tissue and in the cardiovascular system after nephrectomy (Westenfeld R et al., Nephrol Dial Transplant. 2007;22(6):1537-1546) and especially in combination with apolipoprotein E deficiency and nephrectomy (Westenfeld R et al., J Am Soc Nephrol. 2009;20(6):1264-1274). Fetuin-A serum levels are low in patients with uraemia associated soft tissue calcifications suggesting depletion and down-regulation during active calcification (Stenvinkel P et al., Kidney Int. 2005;67(6):2383-2392; Hermans M et al., Kidney Int. 2007;72:202-207; Ketteler M et al., Lancet. 2003;361(9360):827-833.). Immunolocalization studies have invariously detected fetuin-A in calcified lesions of calcified medial of Mönckeberg atherosclerosis (Shanahan C et al., Circulation. 1999;100(21):2168-2176) as well as in calcified atherosclerotic intima lesions (Moe SM et al., Kidney Int. 2005;67(6):2295-2304) confirming the high affinity of this protein for mineral and bone-like apatite mineral (Triffitt JT et al., Nature. 1976;262:226-227).

In summary, there are numerous forms of pathological and non-pathological calcification known in the art and certain chemical factors are known that may have an influence on calcium metabolism and homeostasis.

Today, *in vivo* calcification is mainly detected by X-ray-based techniques, computer tomography (CT) and/or Magnetic Resonance Imaging (MRI) or by fluorescent low-molecular weight compounds that interact with calcium such as, e.g., the OsteoSense^{™} dyes (e.g., OsteoSense^{™}680 or OsteoSense^{™}750) obtained from VisEn Medical, Inc. (Bedford, MA).

However, there is still an unmet need of diagnostic tools to detect calcification *in vivo* in high resolution and/or high sensitivity. In particular, microcalcification is still not satisfactorily detectable by the methods known in the art. In particular, microcalcification of blood vessels such as, e.g., the coronary arteries is still hardly detectable (Lembcke A, Blood Purif 2007;25:115-119).

The present invention relates to a fetuin polypeptide or a functional derivative thereof for use in a method for diagnosis of calcification in a patient.

Fetuins are a family of proteins known in the art. Fetuins are polypeptides of the cystatin superfamily and may, therefore, have comparable molecular structure and properties (Lee C, et al., Front Biosci. 2009.14:. 2911-2922).

Fetuins may be e.g. fetuin-A or fetuin-B.

In the context of the present invention (see Example), it has been surprisingly found that fetuin-A, a calcium-binding protein, after administration to a calcified mouse, is found in calcified lesions and is a better diagnostic reagent for diagnosing pathological calcification than OsteoSenseTm from VisEn Medical Inc..

As used throughout the invention, the term "polypeptide" may be understood as a linear or branched polymer mainly composed of amino acids. A polypeptide may be a small peptide of at least four amino acids or may be a protein. The polypeptide may be composed of natural amino acids and/or artificial amino acids (e.g., beta amino acids, D-amino acids, methylated amino acids, etc.). Furthermore, the amino acid backbone may be modified (e.g., by methylation, alkylation, oxidation, cyclization, dehydratation).

The polypeptide may be provided by any means known in the art. It may be provided by a commercial or a non-commercial supplier, may be provided by a biological sample (in particular a blood sample), may be provided by cell culture or tissue culture, may be provided by any biotechnological techniques known in the art or may be provided by chemical means such as peptide synthesis (e.g., solid phase peptide synthesis).

The fetuin polypeptide may be the full-length fetuin, a truncated fetuin or an elongated fetuin.

As used in the context of the present invention, the term "truncated fetuin" may be understood as a polypeptide comprising or consisting of a part of the full-length fetuin. Preferably, the part of the fetuin polypeptide may have a length of 5, 10, 20, 50, 70, 90, 100, 130, 150, 200 or 300 amino acids. Preferably, the truncated fetuin covers a part or the complete sequence of the N-terminal D1 domain of fetuin (see below and Schinke T et al., J Biol Chem. 1996;271:20789-20796)

Throughout the invention, the term "elongated fetuin" may be understood as a fetuin polypeptide comprising in addition to the full-length fetuin additional amino acids.

The fetuin polypeptide used according to the invention is capable of binding to calcium, preferably with a dissociation constant (Kd) of less than 1000 µM, more preferably with a Kd of less than 500 µM, even more preferably with a Kd of less than 250 µM, even more preferably with a Kd of less than 100 µM, more preferably with a Kd of less than 50 µM and most preferably with a Kd of less than 25 µM. The Fetuin polypeptide may also comprise more than one binding sites for calcium, a calcium salt and/or a calcium cation. The binding may than be independent from another, cooperatively or uncooperatively.

In the context of the present invention the term "calcium" may preferably refer to calcium cations and calcium salts such as calcium phosphate, calcium hydrogen phosphate and/or calcium dihydrogen phosphate, hydroxylapatite and/or to fluoroapatite. Alternatively and additionally, calcium may be present in a mixed crystal comprising calcium salts, such as, e.g., depositions in the vessels comprising calcium salts in combination with other salts, proteins, fatty acids, triglycerides, cholesterols, low density lipoprotein (LDL), high density lipoprotein (HDL) and/or sugars.

The fetuin polypeptide may be derived from any species including human, mouse, rat, bovine and ovine.

Preferably, the fetuin is derived from human.

The present invention also relates to a functional derivative of a fetuin polypeptide. As used herein, the term "functional derivative" refers to polypeptides with a sequence identity of more than 50%, preferably of more than 60%, preferably of more than 80%, more preferably more than 85%, even more preferably more than 90%, even more preferably more than 95%, most preferably more than 97% identity to a fetuin polypeptide and/or at least more than 10%, more than 20%, more than 30%, more than 40%, more than 50%, more than 60%, more than 70%, more than 80%, more than 90%, more than 95%, more than 97% or more than 100 % of the binding affinity to calcium.

A functional derivative of a fetuin polypeptide may be a mutated form of fetuin. A mutated form may be a fetuin polypeptide in which one or more amino acid residues have been inserted, removed and/or exchanged. Such mutated form of fetuin may refer to but may not be limited to fetuin encoded by an altered DNA or RNA sequence, a splice variant of fetuin or fetuin from another species (e.g., a viral fetuin or a bacterial fetuin). An altered DNA or RNA sequence encoding for fetuin may occur naturally throughout the population tested or may be of artificial origin. Alternatively, the functional derivative of a fetuin polypeptide may be of synthetical origin, i.e. chemically modified or synthesized.

Moreover, the functional derivative of a fetuin polypeptide may also be a product of one or more posttranslational modifications. Posttranslational modifications are well-known in the art and may comprise but may not be limited to lipidation, phosphorylation, sulfatation, glycosylation, truncation, cyclization of several amino acid moieties, cyclization of the polypeptide strand, oxidation, reduction, decarboxylation, acetylation, amidation, deamidation, disulfide bond formation, pyroglutamate formation, amino acid addition, cofactor addition (e.g., biotinylation, heme addition) and complexation of metal ions, non-metal ions, peptides or small molecules and addition of iron-sulphide clusters. Evidently, co-factors, such as the binding of ATP, ADP, NAD+, NADH+H+, NADP+, NADPH+H+, metal ions, anions, lipids, etc. may be bound to the polypeptide, irrespective on the biological influence of these co-factors.

According to the invention, the fetuin polypeptide or functional derivative thereof is used for the diagnosis of calcification. The term "calcification" as used herein refers to the deposition and precipitation of insoluble or poorly soluble calcium salts *in vivo.* In the sense of the present invention, the terms "calcification", *"in vivo* calcification" and "calcified lesion" are used interchangeably.

Calcification may refer to the mineralization of the bones and/or the teeth. Here, the calcium cations are mainly incorporated in hydroxylapatite. Calcium is deposited, when the bone grows in the length and/or in the width. Furthermore, lifelong, calcium is permanently incorporated in the bone to maintain the stability. Here, different diseases may lead to a lack of calcification, in particular in elderly people, in more particular in elderly women. Moreover, upon healing of a fracture of a bone calcification may play an important role. In the teeth, a lack of calcification may lead to a lack of physical stability and diminished capability of resistance against caries.

Furthermore, calcification may occur in the lumen of certain organs such as the kidney, in particular in the renal pelvis, the ureter, the bladder, the gallbladder and/or the bile duct. As a result, a kidney stone (nephrolith) and a gallstone, respectively, may occur. Kidney stones and gallstones frequently occur throughout the population and may lead to severe symptoms.

Moreover, calcification may occur in soft tissues and may cause soft tissues to harden. Here; calcification may lead to a pathological tissue and potentially to a corresponding pathological condition including potentially severe clinical symptoms. Several types of tumours such as certain types of brain tumours bear a distinctive local calcification (calcium spots), though calcium spots of different sizes may also occur in the brain without provoking any clinical symptoms or indicating any pathological condition.

Cardiovascular calcification, thus, the formation of calcified lesions in the cardiovascular system, may increase the risk of numerous diseases such as hypertonia, myocardial infarction and cerebral apoplexy (stroke). Highly pronounced forms of calcification may further lead to the necessity of amputation of an extremity such as a leg. One severe form of pathological cardiovascular calcification is "Mönckeberg's atherosclerosis", also called "medial calcific sclerosis". Here, the vessels harden as calcium deposits form in the middle layer of the walls of medium sized vessels *(tunica media).* Consequently, the pulse pressure is pathologically increased.

As used in the context of the present invention, the term "patient" may be understood in the broadest sense as a subject the fetuin polypeptide is administered to, irrespective, whether it is a human or an animal and whether clinical symptoms occur or do not occur. Preferably, the patient is a human patient.

The fetuin polypeptide may be administered to the patient by any means. Preferably, the polypeptide is injected into the tissue or into a blood vessel via a syringe or a drip. Alternatively it may also be injected intraperitoneal or it may be administered orally, nasally, topically or subcutaneously.

In a preferred embodiment, the fetuin is fetuin-A.

In the context of the present invention, the terms "fetuin-A", "alpha2-HS glycoprotein", "AHSG", "Ahsg", "α2-HS", "α2-HS glycoprotein", "A2HS", "AHS" and "HSGA" are interchangeable.

In a preferred embodiment, human fetuin-A has a sequence of (SEQ ID NO:1):

MKSLVLLLCLAQLWGCHSAPHGPGLIYRQPNCDDPETEEAALVAIDYINQNLPWG
YKHTLNQIDEVKVWPQQPSGELFEIEIDTLETTCHVLDPTPVARCSVRQLKEHAVE
GDCDFQLLKLDGKFSVVYAKCDSSPDSAEDVRKVCQDCPLLAPLNDTRVVHAAK
AALAAFNAQNNGSNFQLEEISRAQLVPLPPSTYVEFTVSGTDCVAKEATEAAKCN
LLAEKQYGFCKATLSEKLGGAEVAVTCMVFQTQPVSSQPQPEGANEAVPTPVVDP
DAPPSPPLGAPGLPPAGSPPDSHVLLAAPPGHQLHRAHYDLRHTFMGVVSLGSPSG
EVSHPRKTRTVVQPSVGAAAGPVVPPCPGRIRHFKV

In a preferred embodiment, mouse fetuin-A has a sequence of (SEQ ID NO:2):

MKSLVLLLCFAQLWGCQSAPQGTGLGFRELACDDPEAEQVALLAVDYLNNHLLQ
GFKQVLNQIDKVKVWSRRPFGVVYEMEVDTLETTCHALDPTPLANCSVRQLTEH
AVEGDCDFHILKQDGQFRVMHTQCHSTPDSAEDVRKLCPRCPLLTPFNDTNVVHT
VNTALAAFNTQNNGTYFKLVEISRAQNVPLPVSTLVEFVIAATDCTAKEVTDPAKC
NLLAEKQHGFCKANLMHNLGGEEVSVACKLFQTQPQPANANAVGPVPTANAALP
ADPPASVVVGPVVVPRGLSDHRTYHDLRHAFSPVASVESASGETLHSPKVGQPGA
AGPVSPMCPGRIRHFKI

In a preferred embodiment, bovine fetuin-A has a sequence of (SEQ ID NO:3):

MKSFVLLFCLAQLWGCHSIPLDPVAGYKEPACDDPDTEQAALAAVDYINKHLPRG
YKHTLNQIDSVKVWPRRPTGEVYDIEIDTLETTCHVLDPTPLANCSVRQQTQHAVE
GDCDIHVLKQDGQFSVLFTKCDSSPDSAEDVRKLCPDCPLLAPLNDSRVVHAVEV
ALATFNAESNGSYLQLVEISRAQFVPLPVSVSVEFAVAATDCIAKEVVDPTKCNLL
AEKQYGFCKGSVIQKALGGEDVRVTCTLFQTQPVIPQPQPDGAEAEAPSAVPDAA
GPTPSAAGPPVASVVVGPSVVAVPLPLHRAHYDLRHTFSGVASVESSSGEAFHVG
KTPIVGQPSIPGGPVRLCPGRIRYFKI

In a preferred embodiment, the fetuin polypeptide is the mature fetuin polypeptide, in case of human fetuin-A the sequence according to SEQ ID NO:1 where the amino acids 1-18 have been cleaved off.

In the context of the present invention, the terms "fetuin-B" and "FETUB" are interchangeable.

In a preferred embodiment of the present invention, the fetuin polypeptide is a fetuin calcium-binding domain, in particular the D1 domain of fetuin-A.

Preferably, the fetuin polypeptide comprises or has the following sequence (SEQ ID NO:4):
EGDCDFQLLK.

The term "fetuin calcium-binding domain" as used herein refers to a functional subunit of fetuin that is able to bind calcium with high affinity, thus preferably with a dissociation constant (Kd) of less than 1000 µM, more preferably with a Kd of less than 500 µM, even more preferably with a Kd of less than 250 µM, even more preferably with a Kd of less than 100 µM, more preferably with a Kd of less than 50 µM and most preferably with a Kd of less than 25 µM. The calcium-binding domain of fetuin may comprise one or more than one binding sites for calcium.

In an even more preferred embodiment of the present invention, the fetuin calcium-binding domain is the D1 domain of fetuin-A. The D1 domain is an N-terminal domain of fetuin-A that has a cystatine-like structure (Schinke T et al., J Biol Chem. 1996;271:20789-20796). In a preferred embodiment, the fetuin calcium binding domain comprises or consists of amino acids 5 to 115 of the human mature fetuin-A polypeptide (the protein of SEQ ID NO: 1 where amino acids 1 to 18 have been cleaved off (see also Heiss A et al., J Biol Chem 2003;278:13333-13341). Alernatively, the fetuin calcium binding domain may also comprise or consist of the D1 domain of the mouse or bovine fetuin-A. The computer-modelled domain structures of the D1 domain suggest a dense array of acidic residues on an extended β-sheep (Heiss A et al., J Biol Chem 2003;278:13333-13341). In the context of the present invention, the term "D1 domain of fetuin-A" is used interchangeably with "Ahsg domain D 1 ", "D1 domain" and "D1".

The fetuin polypeptide or functional derivative thereof may be conjugated or bound to other molecules that may preferably refer to but may not be limited to fusion proteins (e.g., fluorescent proteins), one or more lipids or lipidoids, one or more small molecule dyes (e.g., fluorescent dyes or UV/Vis dyes (e.g., a p-nitrophenyl moiety, Coomassie Brilliant Blue G-250), one or more quantum dots, one or more binding moieties (e.g., biotin, methotrexate, glycocorticoids or moieties comprising, e.g., one or more active esters, isothiocyanates, maleimids, N-hydroxysuccinimidyl esters, glutaraldehyde derivatives, carbodiimide derivatives or combinations thereof), one or more insoluble and/or soluble polymers (e.g., polyethylene glycol (PEG), hydroxypropyl methacrylate (HPMA), polyethylene imine (PEI)), one or more antibodies or derivatives thereof (e.g., Fab fragments, single chain antibodies, diabodies, triabodies, flexibodies, tandabs), one or more peptides (e.g., cell-penetrating peptides (CPPs), protein transduction domains (PTDs), signal transduction peptides, etc.), one or more spin labels, one or more enzyme labels (e.g., penicillinase, horseradish peroxidase, alkaline phosphatase), micro- or nanobeads (e.g., functionalized silica beads, polysaccharide-based beads), polymersomes and/or liposomes.

In a preferred embodiment of the present invention, the fetuin polypeptide is labelled, in particular labelled with a fluorescent dye or a luminescent dye.

In the context of the present invention, the term "labelled" may be understood in the broadest sense as a fetuin polypeptide that carries a moiety that enables the detection and/or localisation of the fetuin polypeptide *in vivo.* As used herein the terms "label", "detectable moiety" and "marker" may be used interchangeably. The label may be, e.g., a radioactive label, spin label, a fluorescent label or a luminescent label. The label may be conjugated to the fetuin polypeptide directly or via a functional linker, (e.g., a peptide linker, a polyethylene glycol (PEG) linker, a saccharide linker, a fatty acid linker, an alkyl linker, etc). Alternatively, one or more labelled antibodies or derivatives thereof (e.g., Fab fragments, single chain antibodies, diabodies, triabodies, flexibodies, tandabs) may be labelled and bound to the fetuin polypeptide. The binding to the polypeptide may occur *in vitro* or *in vivo.* Aforementioned labels may be added to the protein in vitro or directly in the patient in vivo.

In the context of the present invention, a radioactive label may include but may not be limited to ³H , ³²P, ³⁵S, ¹⁴C, ^{99m}Tc or lanthonoids (e.g., ⁶⁴Gd) suitable for scintillation assays, computer tomography (CT) or a label suitable for Positron Emission Tomography (PET). The fetuin polypeptide may be directly labelled radioactively or via the conjugation or complexation of a radioactively labelled molecule. Moreover, a fetuin polypeptide may be labelled with a spin label, such as one or more heavy isotopes, e.g., ¹³C detectable by Nuclear Magnetic Resonance (NMR).

In an even more preferred embodiment, the label is a fluorescent dye or a luminescent dye.

As used herein, the terms "fluorescent dye", "fluorescence dye" and "fluorophore" may be used interchangeably. A fluorescent dye may be a fluorescent polypeptide (e.g., cyan fluorescent protein (CFP), green fluorescent protein (GFP) or yellow fluorescent protein (YFP), red fluorescent protein (RFP), mCherry, etc.), a small-molecule dye (e.g., a Cy dye (e.g., Cy3, Cy5, Cy5.5, Cy 7), an Alexa dye (e.g., Alexa Fluor 488, Alexa Fluor 546, Alexa Fluor 647, Alexa Fluor 680, Alexa Fluor 750), a Visen dye (e.g. VivoTag680, VivoTag750), an S dye (e.g., 50387), a DyLight fluorophore (e.g., DyLight 750, DyLight 800), an IRDye (e.g., IRDye 680, IRDye 800), a fluorescein dye (e.g., fluorescein, carboxyfluorescein, fluorescein isothiocyanate (FITC)), a rhodamine dye (e.g., rhodamine, tetramethylrhodamine (TAMRA)) or a HOECHST dye) or a quantum dot. One or more dyes may be combined.

As used herein, the term "luminescent dye" may refer to every molecule that emits light upon a chemical or a biochemical reaction.

In an even more preferred embodiment, the fetuin polypeptide is labelled with a dye that bears an emission maximum at a wavelength between 300 nm and 1500 nm, in more particular between 400 nm and 1000 nm, in even more particular between 450 nm and 800 nm, in even more particular between 450 nm and 750 nm and most particular between 450 nm and 700 nm.

Alternatively, the dye may emit fluorescence in the infrared spectrum for tissue penetration of both the exciting and emitted light, thus, bears an emission maximum at a wavelength above 800 nm, preferably above 1000 nm, more preferably above 1200 nm, even more preferably above 1500 nm.

For the diagnosis of calcification, the fetuin localized at the calcified lesion shall be detected. This detection can be performed by any method known in the art.

The fetuin and thereby the calcification may be detected by means of fluorescent microscopy, Fluorescenct Molecular Tomography (FMT), PET, Single Photon Emission Computed Tomography (SPECT), Fluorescence Molecular Imaging (FMI), X-ray, scintigraphy as known in the art (see, e.g., Fine A and Zacharias J, Kidney Int. 2002;61:2210-2217).

In a preferred embodiment, the fetuin and thereby the calcification is detected by detecting the fetuin label. Preferably, and depending on the nature of the label, Fluorescence Molecular Imaging (FMI), in particular Fluorescence Molecular Tomography (FMT) is used for detecting calcification.

As used herein, the term "Fluorescence Molecular Imaging (FMI)" may be understood in the broadest sense as a technique to detect the localization of fluorescently labelled molecules *in vivo* and *in vitro.* Numerous techniques based on FMI are known to those skilled in the art. Preferably, the result obtained by FMI is displayed visually and graphically. Alternatively, the local brightness may be displayed quantitatively as a number.

As used herein, the term "Fluorescence Molecular Tomography (FMT)" may be understood as a technique based on FMI, wherein the localisation of the fluorescently labelled molecules is detected in a three-dimensional space (3D). This may be accomplished by revolving either the light emitter or the detector around the subject of interest. Alternatively, the subject may be revolved against the emitter and detector. In yet another setup, the system may be based on a two photon system. Here, long wavelengths, thus low-energy photons are used. Therefore, the molecule of interest is only excited when two photons hit it within a very short time interval. In a particular setup, the emission light of a long wavelength may be emitted into the sample in a cone-shape. Only at the cone end the photon density is high enough to excite the molecules. Therefore, only a locally distinct area in the subject emits light. By moving the cone, the whole sample may be scanned in a 3D manner and subsequently a computer may generate a 3D image of the subject. Alternatively, the two-photon system may be based on two emitters of which one or both may emit light in either an optical plane or a light beam or a cone. Then, only the intersections of the beams emit light. Furthermore, systems based on three or even more photons may be used.

Alternatively, other molecular imaging techniques such as, e.g., Magnetic Resonance Imaging (MRI), Optical Imaging, Single Photon Emission Computed Tomography (SPECT), Positron Emission Tomography (PET), Ultrasound or Computer Tomography (CT) or combinations thereof may be used. These techniques are well-known to those skilled in the art.

In a preferred embodiment of the present invention, the calcification is a microcalcification.

As used herein, the term "microcalcification" refers to tiny specks of calcium-containing mineral deposits, that may be scattered throughout the body, in particular the soft tissues. These "specks" may also be designated as "clusters" or "spots". In contrast to the mineralization of bones and teeth, microcalcification is characterized by smaller volumes. Microcalcification may occur upon the growth of a tumour, e.g., a tumour in the brain or in the mammary gland. Furthermore, microcalcification may be a deposit in a vessel or in a lumen of an inner organ.

In a more preferred embodiment, the calcification is a pathological mineralization.

As used in the context of the present invention, the term "pathological mineralization" refers to all kinds of mineralization comprising calcium that are not physiological. The term "pathological" does not necessarily indicate that clinical symptoms occur, though clinical symptoms may occur. A pathological mineralization may be a result of a disease or it may be a result of a metabolic dysfunction or result from false alimentation. But, pathological mineralization may also occur in healthy subjects, in particular in elderly subjects.

In an even more preferred embodiment, the patient is suffering from atherosclerosis, in particular atherosclerosis in a patient suffering from (i) nephropathy, (ii) calciphylaxis, (iii) Mönckeberg's atherosclerosis, (iv) hypertonia and/or (v) cancer.

As used herein, the terms "atherosclerosis", "arteriosclerosis", "cardiovascular atherosclerosis", "cardiovascular arteriosclerosis" and "cardiovascular plaque" may be used interchangeably in the broadest sense as the deposition of plaque in the blood vessels. The plaque may be deposited in the lumen of the blood vessels or in the in the middle layer of the walls of medium sized vessels *(tunica media).* The plaque may comprise calcium salts in combination with other minerals, proteins, fatty acids, triglycerides, cholesterols, low density lipoprotein (LDL), high density lipoprotein (HDL) and/or sugars. Furthermore, cells or cellular fragments such as, e.g., macrophages, red blood cells (RBCs) and platelets, may be part of the plaque.

Pathological forms of atherosclerosis may be also designated as arteriosclerotic vascular disease or (ASVD). Cardiovascular plaque may increase the risk of numerous diseases such as hypertonia, myocardial infarction and cerebral apoplexy (stroke). Highly pronounced forms of calcification may further lead to the necessity of amputation of an extremity such as a leg.

One of the most pronounced from of atherosclerosis is Mönckeberg's atherosclerosis. The terms "Mönckeberg's atherosclerosis", "Monckeberg's atherosclerosis" and "medial calcific sclerosis" may be used interchangeably. Mönckeberg's atherosclerosis is one of the most severe forms of atherosclerosis. Here, the vessels harden as calcium deposits form in the middle layer of the walls of medium sized vessels *(tunica media).*

The term "nephropathy" as used herein refers to a dysfunction or a non-function of one or both kidneys. Calciphylaxis is a severe syndrome of vascular calcification, thrombosis and skin necrosis. The term "hypertonia" as used herein refers to a condition high blood pressure and is well-known in the art. The term "cancer" may be understood in the broadest sense. It may include the occurrence primary and secondary tumours, metastases and other kinds of pathological neoplasms.

In another preferred embodiment of the present invention, the pathological mineralization is provoked by fetuin deficiency, in particular fetuin-A deficiency.

As used herein, the term "fetuin deficiency" refers to a lower systemic level of fetuin in the patient. This lower level may be based on a lower expression level of fetuin or on an accelerated degradation of fetuin. It may have a genetic reason or may be caused by pathogens or may be the result of a down regulation of the expression level by pharmacological compounds, xenobiotics and/or interfering RNA. As used herein, the term "fetuin-A deficiency" refers to a lower systemic level of fetuin-A in the patient.

In a preferred embodiment of the present invention, the pathological mineralization is occurring in one or more soft tissues, in particular in the skin, brown fat, lung, kidney, heart and/or in a joint. In another preferred embodiment of the present invention, the patient suffers from a rheumatoid disease.

In the context of the present invention, a rheumatoid disease may be understood in the broadest sense as an inflammatory disease of a joint. It may be the result of an autoimmune response and/or of pathological mineralization of the joint. It may be a mixed crystal of several salts, e.g., calcium, bile acids, uric acid and/or different proteins.

In another preferred embodiment of the present invention, the fetuin polypeptide is used in a method for diagnosis of calcification in bones, in particular when the bone metabolism is pathologic.

In the context of the present invention, pathologic bone metabolism may refer to a lack of calcification in bones or a pathologic condition in which the calcium concentration in the bone is too high and the organic material is too less. Furthermore, pathologic bone metabolism may be dwarfism (hyposomia), gigantism (hypersomia), low bone turnover, osteomalacia, osteofibrosis.

Another aspect of the present invention relates to a diagnostic kit comprising the fetuin polypeptide as defined above and optionally further comprising (i) one or more pharmaceutically acceptable carriers and/or (ii) a user manual.

In the context of the present invention, the term "kit" may be understood in the broadest sense as a composition of different products that may be used for performing a diagnosis. The kit may comprise, but may not be limited to the fetuin polypeptide or a functional derivative thereof, pharmaceutically acceptable carriers, a user manual, syringes, needles, etc. Optionally, the fetuin polypeptide or functional derivative thereof may be dissolved or may be freeze-dried. As used herein, the term "pharmaceutically acceptable carrier" may refer to any substance that may support the pharmacological acceptance of the fetuin polypeptide. The fetuin polypeptide may be administered orally or may be injected. It may be pharmaceutically formulated in a dry form (e.g., as a powder, a tablet, a pill, a capsule, a chewable capsule, etc.) or a liquid (e.g., a spray, a syrup, a juice, a gel, a liquid, a paste, an injection solution, etc.) A pharmaceutically acceptable carrier may be a solvent with no or low toxicity such as, e.g., water, dimethyl sulphoxide (DMSO), ethanol, vegetable oil, paraffin oil or combinations thereof. Furthermore, the pharmaceutically acceptable carrier may contain one or more detergents, one or more foaming agents (e.g., sodium lauryl sulfate (SLS)/ sodium doceyl sulfate (SDS)), one or more colouring agents (e.g., TiO₂ food colouring), one or more vitamins, one or more salts (e.g., sodium, potassium, calcium, zinc salts), one or more humectants (e.g., sorbitol, glycerol, mannitol, propylenglycol, polydextrose), one or more enzymes, one or more preserving agents (e.g., benzoic acid, methylparabene), one or more texturing agents (e.g., carboxymethyl cellulose (CMC), polyethylene glycol (PEG), sorbitol), one or more emulsifiers , one or more bulking agents, one or more glacing agents, one or more separating agents, one or more antioxidants, one or more herbal and plant extracts, one or more stabilizing agents, one or more polymers (e.g., hydroxypropyl methacrylamide (HPMA), polyethylene imine (PEI), carboxymethyl cellulose (CMC), polyethylene glycol (PEG)), one or more uptake mediators (e.g., polyethylene imine (PEI), dimethyl sulphoxide (DMSO), a cell-penetrating peptide (CPP), a protein transduction domain (PTD), an antimicrobial peptide, etc.) one or more antibodies, one or more sweeteners (e.g., sucrose, acesulfam K, saccharin Na), one or more counterstain dyes (e.g., fluorescein, fluorescein derivatives, Cy dyes, an Alexa Fluor dyes, S dyes, rhodamine, quantum dots, etc.), one or more homeopathic ingredients one or more gustatory substances and/or one or more fragrances.

Preferably, the kit of the invention includes a freeze-dried labelled fetuin-A popolypeptide, a buffer to dissolve the fetuin-A polypeptide, an injection or infusion device to mix the polypeptide with the buffer and to administer the mixture to a patient.

The invention further relates to a method for the diagnosis of calcification, wherein a fetuin polypeptide or a functional derivative thereof is administered to a patient and the localisation of said fetuin polypeptide or functional derivative thereof at the calcified lesions is detected. All embodiments defined above with respect to the use of the invention also apply to this method of the invention.

The following figures and example are intended to illustrate the invention but not to limit the scope of protection conferred by the claims.

### Figures

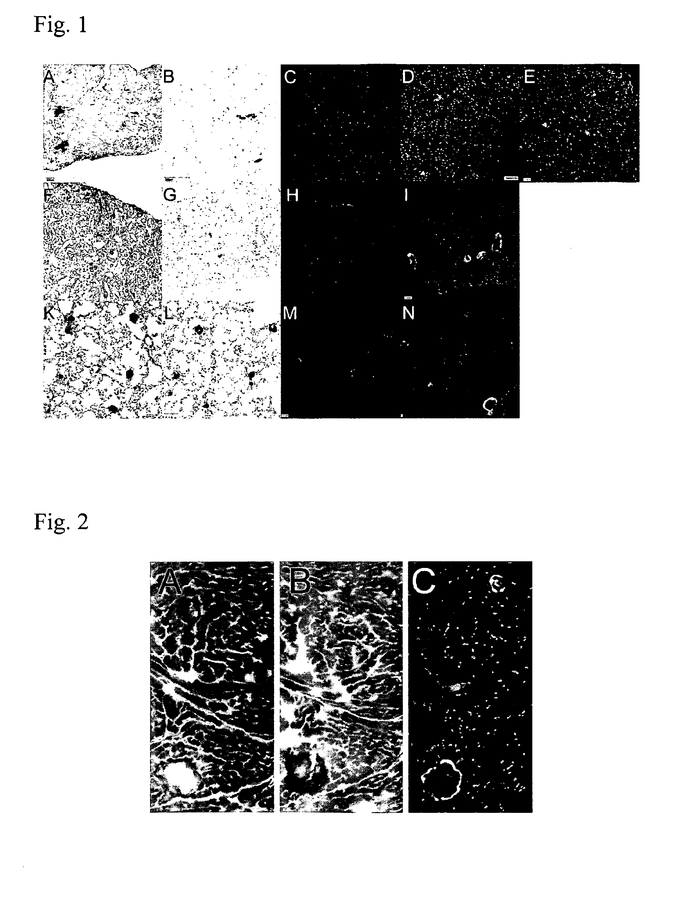

**Figure 1****.** Staining calcified lesions in fetuin-A deficient mice. A-E myocard, F-J kidney and K-O lung. A, F, K Hematoxilin/Eosin stained sections showed large lesions in myocard (A) and lung (K) that failed to stain in the kidney (F). B, G, L von Kossa staining demonstrated calcified lesions in myocard (A), lung (K) and kidney (F). C, H, M show calcified lesions in a mouse injected with a single bolus of unlabelled fetuin-A. Bound fetuin-A was detected using rabbit anti fetuin antibody and red fluorescent secondary antibody. D, I, N depict cryosections from a mouse injected with a single bolus of Alexa Fluor® 488-labelled fetuin-A. E, J, O show calcified lesions similar to those shown in D, I, N, but prepared from a non-injected mouse. The sections were post-stained with Alexa Fluor® 488-labelled Fetuin-A. Note that established histology methods detected only large calcified lesions whereas fetuin-A, unlabelled, or Alexa Fluor® 488-labelled, injected into live animals or applied to histological sections also detected much smaller lesions. Size bars depict 200 µm.
**Figure 2****.** Serial sections of the heart from a fetuin-A deficient mouse. Hematoxylin-Eosin staining showed only a big lesion (A), von Kossa staining could also detect a smaller lesion in the upper part of the section (B), whereas injection of Alexa Fluor® 488-labelled fetuin-A enabled the detection of an additional very small lesion (C).

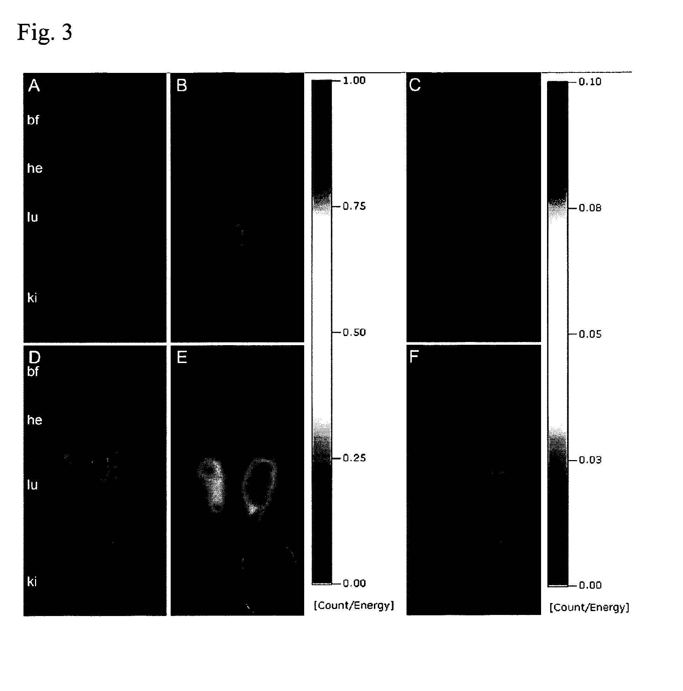

**Figure 3****.** Explanted organ fluorescence 2D in reflectance imaging. DBA/2 fetuin-A deficient mice (D, E, F) or wildtype mice (A, B, C) were co-injected with one bolus each of OsteoSense 750® and Alexa Fluor® 680 labelled fetuin-A. Animals were sacrificed and organ fluorescence was recorded in 2D-mode (planar reflectance). Alexa Fluor®-labelled fetuin-A fluorescence is depicted in B, D and F, OsteoSense 750® fluorescence is depicted in A, B and C. Alexa Fluor® 680 labelled fetuin-A strongly stained brown fat (bf), lungs (lu) and kidneys (ki), which were heavily calcified in the fetuin-A deficient mice (D, E, F), but not in wildtype mice (A, B, C). The heart (he) stained weakly. Using the identical tissues and signal amplification instrument settings OsteoSense 750® stained negative in tissues that were brightly stained by Alexa Fluor® 680 labelled fetuin-A (A, D vs. B, E). At 10-fold stronger signal amplification OsteoSense 750® depicted brown fat and lungs in the fetuin-A deficient mouse (F), yet still much weaker than Alexa Fluor® 680 labelled fetuin-A.

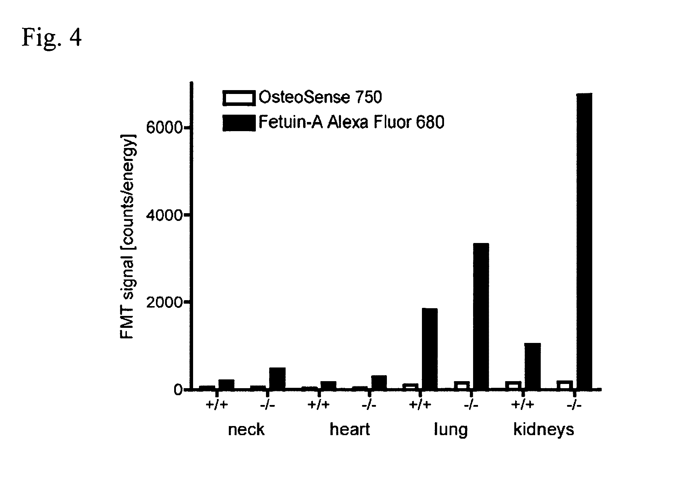

**Figure 4****.** FMT signal strength in organs of wildtype and fetuin-A deficient DBA/2 mice. Shown is the fluorescence quantification of brown fat in the neck, heart, lung and kidney of both OsteoSense 750® and Alexa Fluor® 680 labelled fetuin-A in counts per energy. Note that lung and kidneys stained with fetuin-A give the highest signal.

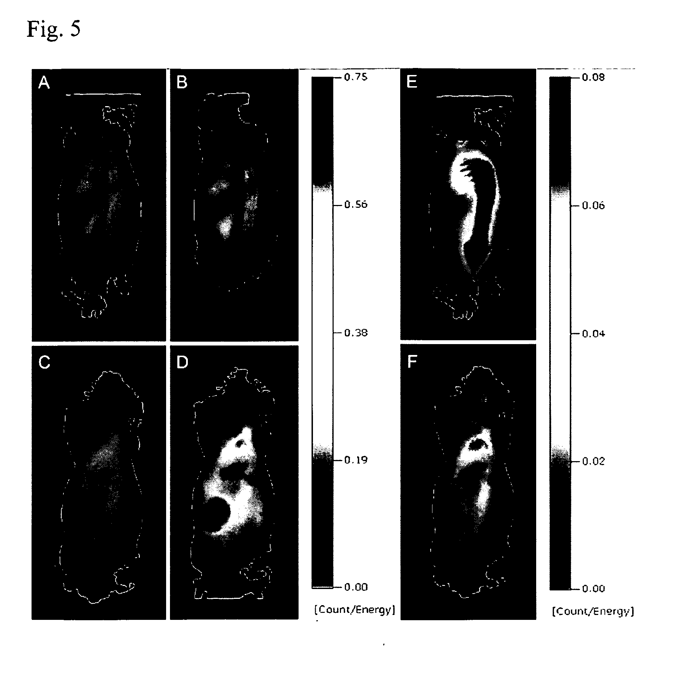

**Figure 5****.** *In vivo* OsteoSense® 750 and Fetuin-A Alexa Fluor® 680 fluorescence molecular tomography in wildtype and Fetuin-A -/- mice. Whole body fluorescence was recorded in 2D-mode. A, B and E show the fluorescence measured in wildtype, C, D and F depict a fetuin-A deficient animal. Both animals received equal amounts of OsteoSense 750® and Alexa Fluor® 680-labelled fetuin-A. The detected signal for the wildtype mouse was low for both agents (A, B, E) whereas the knockout animal showed high fluorescence in the Fetuin-A channel in contrast to the OsteoSense fluorescence (C, D, F). Alexa Fluor®-labelled fetuin-A strongly stained brown fat in the neck and kidneys.

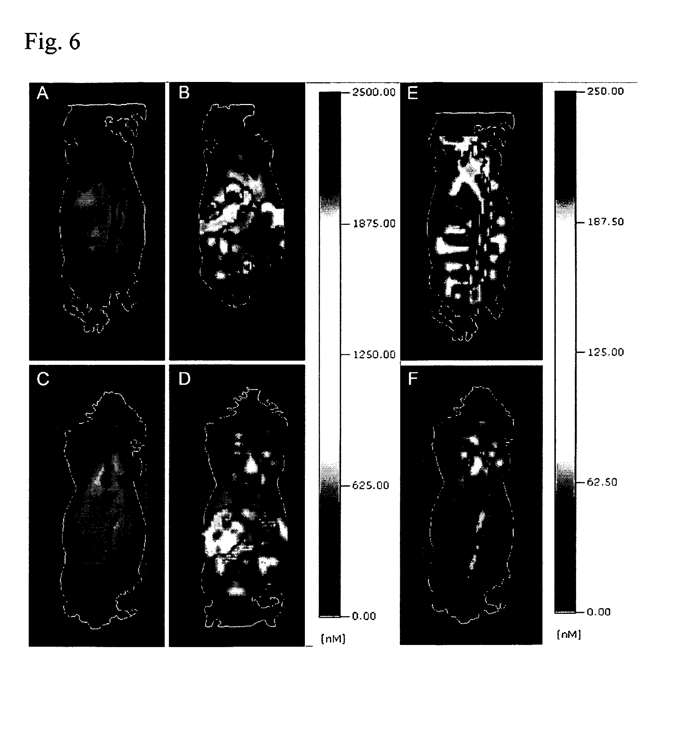

**Figure 6****.** Three-dimensional *in vivo* OsteoSense® 750 and Fetuin-A Alexa Fluor® 680 fluorescence molecular tomography in wildtype and Fetuin-A deficient mice. Whole body fluorescence was recorded in 3D-mode. A, B and E show the fluorescence measured in wildtype, C, D and F depict a Fetuin-A -/- animal. Both animals received equal amounts of OsteoSense 750® and Alexa Fluor® 680-labelled fetuin-A. The fluorescence signal for OsteoSense 750® was low, however it was higher in the wildtype than in the knockout animal (A, C). The Fetuin-A signal was much higher, in the knockout mouse it could be highly detected in the neck and in the left kidney but also in the gut (B, D). At 10-fold stronger signal amplification OsteoSense 750® depicted brown fat and the spine in the fetuin-A deficient mouse (F).

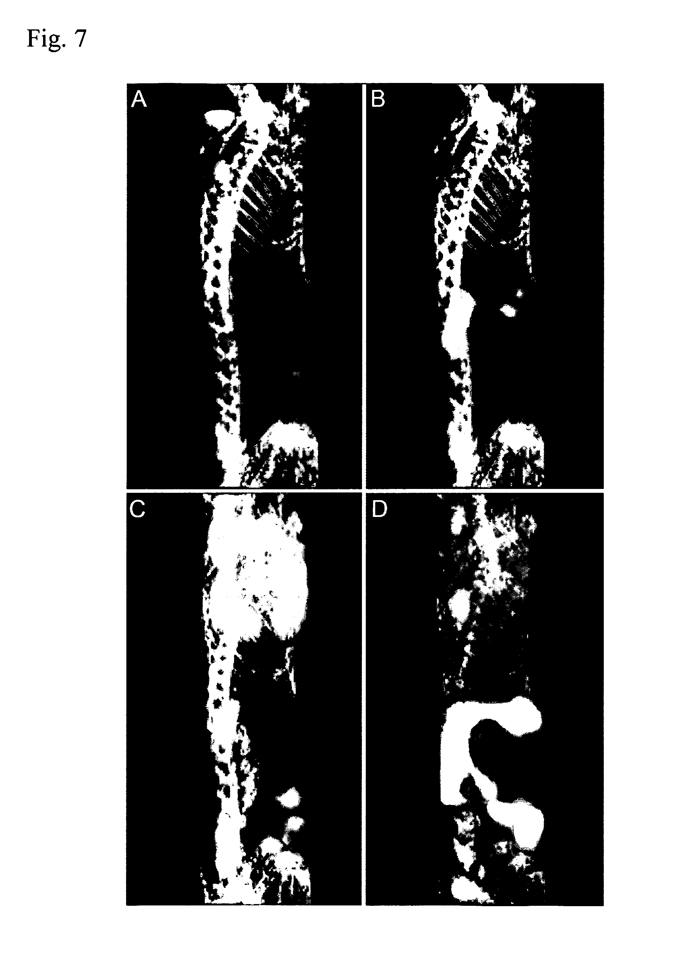

**Figure 7****.** Combined Fluorescence Molecular Tomography and micro computed tomography in wildtype and fetuin-A deficient mice. Whole body fluorescence was recorded in 3D-mode and fused with the corresponding CT-picture with the help of amide software. A and B show a wildtype mouse, C and D depict a fetuin-A deficient animal. Both animals received equal amounts of OsteoSense 750® and Alexa Fluor® 488-labelled fetuin-A. The wildtype animal shows lower signals than the fetuin-A deficient mouse. OsteoSense 750® stained mainly the neck and thorax of the fetuin-A deficient animal, whereas Alexa Fluor® 680-labelled fetuin-A showed the highest signal in the abdomen (namely the kidney and the gut) of the mouse.

### Example

### Experimental Setup

### Fetuin-labelling

Fetuin-A (Sigma Aldrich, F2379) was purified by gel chromatography in phosphate buffered saline (50mM Na-phosphate pH 7.4, 140 mM NaCl) to separate high molecular weight aggregates (10%), fetuin-A monomer (30%), and fragments/salt (60%). Fetuin-A monomer was coupled to Alexa Fluor dyes (Alexa Fluor® 488 or Alexa Fluor® 680, Invitrogen) essentially following the labelling protocol provided by Invitrogen.

### Animals

All animal experiments were conducted in agreement with German Animal Protection Law and were approved by the state animal welfare committee. We used fetuin-A deficient mice maintained on the genetic background DBA/2 (ILAR designation D2, Fetuin-A-/- wjatm1), DBA/2 wildtype mice and apolipoprotein E deficient mice maintained on the genetic background C57BL/6 ApoE -/- mice. Male and female mice were used for this study. The mice were kept in a 12 hours dark /12 hours light cycle in the animal facility of the RWTH Aachen University Clinics and received normal chow (ssniff® R/M-H) and water *ad libitum.* The mice were at least 7 weeks old, at which time the fetuin-A deficient mice show clear soft tissue calcification.

### Histology

The mice were injected with unlabelled or Alexa Fluor® 488 coupled fetuin-A. They received 100 µl / 25 g body weight of a 7 mg/ml solution. Five hours after injection the mice were killed, organs were harvested, embedded in TissueTek (Sakura), and snap-frozen and in liquid nitrogen. Serial cryotome sections were prepared and fixed in ice cold acetone. For general histology hematoxilin/eosin staining and von-Kossa staining were performed.

To localize unlabelled fetuin-A, sections were incubated for one hour with a homemade polyclonal rabbit anti bovine fetuin-A antibody followed by secondary goat anti rabbit Alexa Fluor® 546 antibody and DAPI staining. To localize calcified lesions, sections were incubated with Alexa Fluor® 488-coupled fetuin-A for one hour and then were counterstained with DAPI. Sections of organs from mice injected with Alexa Fluor® 488 labelled Fetuin A were stained with DAPI only.

### FMT-Measurement

We used the FMT^{™} 2500 instrument from VisEn Medical Inc. (Bedford, MA). Mice were were anesthetized with Isofluran (1.5% air 2L/min), depilated in the scanning area and a background measurement was taken for both wavelengths (680 nm and 750 nm). After this procedure the animals received both OsteoSense 750® (2 nmol in a volume of 150 µl) and Fetuin-A Alexa 680 (50 µl of a 7mg/ml solution) by intraperitoneal injection. 24 h after this application, the mice were analyzed in the FMT again and the fluorescence intensity was detected. Reconstruction of the data was performed by VisEn software.

### µCT Scan Protocol

A dual energy µCT for small animals (TomoScope DUO, CT-Imaging, Erlangen, Germany) was used for anatomical image acquisition. The two electron tubes were both run at energies of 65KV. Each flat panel detector acquired 720 projections at 5 fps, containing 1032x1012 pixels with a pixel size of (50µm)². A full rotation was performed and the scan time was 90 seconds per subscan covering 30.8 mm in the transversal direction. Two subscans were acquired to cover almost the entire mouse body. A Feldkamp algorithm was used for reconstruction with isotropic voxel size of 70m, dimensions of 600x500x440, field of view of 42mm x 35mm x 30.8mm and a soft reconstruction kernel (T10).

### CT-FMT Imaging

The FMT probe was injected 24 hours before imaging through i.v. tail vein injection. Hair was removed with depilatory crème around the region of interest. For preparations and imaging, mice were anesthetized with Isofluran. The mouse was positioned in a custom designed multimodal imaging cartridge (CT-Imaging), which contained holes to be used as fiducial markers for image fusion. After scanning in the µCT, the cartridge with the mouse was removed and inserted into the FMT instrument in the same laboratory. The cartridge held the mouse tightly, avoiding motion during the transfer. Constant flow of narcotic gas during transfer was achieved with a long flexible tube attached to the cartridge.

### CT-FMT Fusion

The reconstructed 3D data sets from CT and FMT were fused by computing a rigid body transformation minimizing the residual error between fiducial markers detected in both modalities. For reproducibility, accuracy and user comfort, a software program was developed for automated marker detection and fusion. The average number of markers that were found in both modalities was 12 with average fiducial error of 0.174mm, which was evaluated on a representative set of 31 scans. The FMT reconstruction was warped to the FMT space and stored to allow visualization and analysis with other programs.

### Results

### Fetuin-A binds to calcified lesions

Figure 1 illustrates typical examples of calcified lesions in myocard (Fig. 1A-E), kidney (Fig. 1F-J) and lung (Fig. 1K-O) in fetuin-A deficient mice maintained on the genetic background DBA/2 (D2, Fetuin-A-/-). At ages 3-5 months the calcified lesions ranged in size from few micrometers (Fig. 1E) to several hundred micrometers (Fig. 1H). Few lesions had small or intermediate size suggesting that calcification proceeded very rapidly once started. We wanted to study early stages and progression of calcification yet were hindered by the lack of adequate probes and staining technique. Thus we employed fetuin-A - both unlabelled with subsequent immunostaining as an indirect stain, and fluorescence-labelled as a direct stain - to visualize calcified lesions (Fig. 1C-E, H-J, M-O). Fluorescence-labelled fetuin-A readily stained calcified lesions both in vivo (Fig. 1D, I, N) and on sections (Fig. 1E, J, O). Fetuin-A also depicted small lesions that were not detected by H/E or von Kossa staining (Fig. 2). Having established fluorescence labelled fetuin-A as a useful stain for calcified lesions we studied the progression of calcification in Fetuin-A-/- in-vivo using fluorescence molecular tomography, FMT.

### Fluorescence Molecular Imaging using Fetuin-A

We employed simultaneous OsteoSense 750 an established bone mineral staining probe and Fetuin-A Alexa 680 to detect calcifications in mice. Figure 3 shows 2D reflectance images of brown fat tissue in the neck (bf), heart (he), lung (lu), kidneys (ki), which are known to calcify in D2, Fetuin-A-/-, but not in wildtype mice. Accordingly, tissues from wildtype mice showed low signal of 0.05 Count/Energy or less with OsteoSense® at both 1-fold and 10-fold sensitivity setting of the instrument (Fig. 3A and C, respectively), and of 0.25 Count/Energy with fetuin-A as a probe (Fig. 3B). Fetuin-A staining was strongest in the lung and in the kidneys of wildtype mice suggesting retention of fetuin-A in these organs even in the absence of any calcification. Figure 4 illustrates the quantification of the combined signals of the organs shown in Fig. 3 that were calculated from regions of interest (ROI) containing the organs. Lungs (1816 counts/energy) and kidneys (1025 counts/energy) showed the highest signals in wildtype mice suggesting that these organs accumulate fetuin-A. The tissue accumulation was almost doubled in lungs (3303 counts/energy) and increased over six-fold in kidneys (6740 counts/energy) of Fetuin-A-/- mice. The increased signals originated from combined tissue retention and staining of calcified lesions. By comparison the signals obtained with OsteoSense® staining were much dimmer at around 100 counts/energy yet with similar tissue distribution like fetuin A.

FMT allows to observe probe distribution in vivo, in live animals. The 2D modality yields an anatomical overview of organs, however does not allow absolute quantification of signals in terms of calibrated probe amount. The method is limited to tissue depths of about 1 cm with inherent large signal variation depending on the optical transparency of the tissue. We determined the distribution of OsteoSense® 750 and fetuin A Alexa 680 stain in calcified D2, Fetuin-A-/- mice.

Figure 5 shows the results of a 2D scan of D2, Fetuin-A+/+ wildtype and of D2, fetuin-A-/- fetuin-A deficient mice. Wildtype mice (Fig.5 A,B) injected with either Osteosense® or Fetuin-A, and D2, fetuin-A-/- mice injected with Osteosense® showed no signal at the sensitivity settings that yielded strong signals when D2, Fetuin-A-/- mice were injected with fetuin-A (Fig. 5D). At tenfold increased sensitivity OsteoSense® depicted the spine in the D2, fetuin-A+/+ mouse and the brown fat pad in the neck of the D2, Fetuin-A-/- mouse (Fig. 5E, F).

Thus, in 2D reflectance mode, both Osteosense® and Fetuin-A probes stained brown fat pad in the neck, and Fetuin-A in addition stained the kidney. Differential kidney retention of fetuin-A but not of Osteosense® suggested binding of fetuin-A to contents of the fluid filled kidney cyst of this hydronephrotic kidney, as well as binding to the damaged kidney parenchyma. Most likely fetuin-A bound the mineral rich concretions found in the cyst fluid, while Osteosense® did not.

Next, we performed a 3D tomography of whole mice, which in principle allows the quantification of probe against a calibrated standard. Figure 6 shows the results of 3D fluorescence tomography of a representative DBA/2 wildtype mouse (Fig. 6A, B, E) and a representative fetuin-A deficient mouse (Fig. 6C, D, F) both simultaneously injected with Osteosense® 750 and fetuin-A Alexa 680. Osteosense staining dimly depicted the spine, gut and thorax (Fig. 6A), which became more apparent at 10-fold increased sensitivity (Fig. 6E). As in the 2D mode, fetuin-A Alexa 680 staining outlined the gut, kidneys and the brown fat pad in the neck (Fig. 6B). Staining of the digestive tract suggested that both Osteosense® 750 and fetuin-A Alexa 680 were at least partially excreted through this route and stained the contents of the gut. This was confirmed when we emptied the gut and determined that the signal remained associated with the faeces. Strong staining of the brown fat pad in the neck of D2, Fetuin-A-/- mice suggested that this tissue was heavily calcified, a finding that was independently verified by histology and radiography (not shown). An attempt was made to co-register the FMT signals with micro computed tomography in wildtype and fetuin-A deficient mice for improved localization of the 3D FMT signals. To this end we fused the image series of FMT and micro-CT using amide software (Fig. 7). Please note that due to the volume rendering method of the amide software, there is no possibility of relative comparison between the signal strength received from Osteosense® and fetuin-A Alexa 680 as shown in Figures 3, 5 and 6.

D2, fetuin-A+/+ wildtype mice injected with Osteosense® (Fig.5 A) showed weak fluorescence colocalized with the spine of the animal suggesting that Osteosense® bound to this part of the body whereas staining with fetuin-A Alexa 680 (Fig.5 B) gave a low signal in the digestive tract of the wildtype mouse. D2, fetuin-A-/- mice injected with Osteosense® (Fig.5 C) showed the highest signal in the neck and in the thorax and a weaker one in the digestive tract, fetuin-A Alexa 680 (Fig. 7 D) gave a faint signal in the neck and the thorax, but a higher one in the kidney and the digestive tract. Those results underlined our findings in Figure 6.

### References

1. Brown WM, Dziegielewska KM, Saunders NR, Christie, DL, Nawaratil P, Müller-Esterl W, The nucleotide and deduced amino acid structures of sheep and pig fetuin. Eur J Biochem. 1992;205:321-331
2. Fine A and Zacharias J, Calciphylaxis is usually non-ulcerating: risk factors, outcome and therapy. Kidney Int. 2002;61:2210-2217
3. Heiss A, DuChesne A, Denecke B, Grötzinger J, Yamamoto K, Renné T, Jahnen-Dechent W. Structural Basis of calcification Inhibition by alpha2-HS Glycoprotein/Fetuin-A. J Biol Chem 2003;278:13333-13341.
4. Hermans M, Brandenburg V, Ketteler M, Kooman J, van der Sande F, Boeschoten E, Leunissen K, Krediet R, Dekker F. Association of serum fetuin-A levels with mortality in dialysis patients. Kidney Int. 2007;72:202-207.
5. Jahnen-Dechent W, Schäfer C, Ketteler M, McKee M. Mineral chaperones: a role for fetuin-A and osteopontin in the inhibition and regression of pathologic calcification. J Mol Med. 2008;86(4):379-389.
6. Ketteler M, Bongartz P, Westenfeld R, Wildberger J, Mahnken A, Bohm R, Metzger T, Wanner C, Jahnen-Dechent W, Floege J. Association of low fetuin-A (AHSG) concentrations in serum with cardiovascular mortality in patients on dialysis: a cross-sectional study. Lancet. 2003;361(9360):827-833.
7. Lee C, Bongcam-Rudloff E, Sollner C, Jahnen-Dechent W, Claesson-Welsh L.Type 3 cystatins; fetuins, kininogen and histidine-rich glycoprotein. Front Biosci. 2009;14:2911-2922
8. Lembcke A. Coronary Artery Calcifications: A Critical Assessment of Imaging Techniques, Blood Purif 2007;25:115-119.
9. Merx M, Schäfer C, Westenfeld R, Brandenburg V, Hidajat S, Weber C, Ketteler M, Jahnen-Dechent W. Myocardial stiffness, cardiac remodeling, and diastolic dysfunction in calcification-prone fetuin-A-deficient mice. J Am Soc Nephrol. 2005;16(11):3357-3364.
10. Moe SM, Reslerova M, Ketteler M, O'neill K, Duan D, Koczman J, Westenfeld R, Jahnen-Dechent W, Chen NX. Role of calcification inhibitors in the pathogenesis of vascular calcification in chronic kidney disease (CKD). Kidney Int. 2005;67(6):2295-2304.
11. Schäfer C, Heiss A, Schwarz A, Westenfeld R, Ketteler M, Floege J, Müller-Esterl W, Schinke T, Jahnen-Dechent W. The serum protein alpha 2-Heremans-Schmid glycoprotein/fetuin-A is a systemically acting inhibitor of ectopic calcification. J Clin Invest. 2003;112(3):357-366.
12. Schinke T, Amendt C, Trindl A, Pöschke O, Müller-Esterl W, Jahnen-Dechent W, The serum protein alpha2-HS glycoprotein/fetuin inhibits apatite formation in vitro and in mineralizing calvaria cells. A possible role in mineralization and calcium homeostasis. J Biol Chem. 1996;271:20789-20796.
13. Shanahan C, Cary N, Salisbury J, Proudfoot D, Weissberg P, Edmonds M. Medial localization of mineralization-regulating proteins in association with Monckeberg's sclerosis: evidence for smooth muscle cell-mediated vascular calcification. Circulation. 1999;100(21):2168-2176.
14. Shanahan C, Proudfoot D, Tyson K, Cary N, Edmonds M, Weissberg P. Expression of mineralisation-regulating proteins in association with human vascular calcification. Z Kardiol. 2000;89 Suppl 2:63-68.
15. Stenvinkel P, Wang K, Qureshi AR, Axelsson J, Pecoits-Filho R, Gao P, Barany P, Lindholm B, Jogestrand T, Heimbürger O, Holmes C, Schalling M, Nordfors L. Low fetuin-A levels are associated with cardiovascular death: Impact of variations in the gene encoding fetuin. Kidney Int. 2005;67(6):2383-2392.
16. Triffitt JT, Gebauer U, Ashton BA, Owen ME, Reynolds JJ. Origin of plasma alpha2HS-glycoprotein and its accumulation in bone. Nature. 1976;262:226-227.
17. Westenfeld R, Schäfer C, Krüger T, Haarmann C, Schurgers LJ, Reutelingsperger C, Ivanovski O, Drueke T, Massy ZA, Ketteler M, Floege J, Jahnen-Dechent W. Fetuin-A protects against atherosclerotic calcification in CKD. J Am Soc Nephrol. 2009;20(6):1264-1274.
18. Westenfeld R, Schäfer C, Smeets R, Brandenburg V, Floege J, Ketteler M, Jahnen-Dechent W. Fetuin-A (AHSG) prevents extraosseous calcification induced by uraemia and phosphate challenge in mice. Nephrol Dial Transplant. 2007;22(6):1537-1546.

## Claims

1. A fetuin polypeptide or a functional derivative thereof for use in a method for diagnosis of calcification in a patient.

2. The fetuin polypeptide according to claim 1, wherein the fetuin is fetuin-A.

3. The fetuin polypeptide according any of claims 1 or 2, wherein the fetuin polypeptide is a fetuin calcium-binding domain, in particular the D 1 domain of fetuin-A.

4. The fetuin polypeptide according to any of claims 1 to 3, wherein the fetuin polypeptide is labelled, in particular labelled with a fluorescent dye or a luminescent dye.

5. The fetuin polypeptide according to any of claims 1 to 4, wherein the calcification is detected by Fluorescence Molecular Imaging (FMI), in particular Fluorescence Molecular Tomography (FMT).

6. The fetuin polypeptide according to any of claims 1 to 5, wherein the calcification is a microcalcification.

7. The fetuin polypeptide according to any of claims 1 to 6, wherein the calcification is a pathological mineralization.

8. The fetuin polypeptide according to claim 7, wherein the patient is suffering from atherosclerosis, in particular atherosclerosis in a patient suffering from (i) nephropathy, (ii) calciphylaxis, (iii) Mönckeberg's atherosclerosis, (iv) hypertonia and/or (v) cancer.

9. The fetuin polypeptide according to claims 7 or 8, wherein the pathological mineralization is provoked by fetuin deficiency, in particular fetuin-A deficiency.

10. The fetuin polypeptide according to any of claims 7 to 9, wherein the pathological mineralization is occurring in one or more soft tissues, in particular in the skin, brown fat, lung, kidney, heart and/or in a joint.

11. The fetuin polypeptide according to any of claims 1 to 10, wherein the patient suffers from a rheumatoid disease.

12. The fetuin polypeptide according to any of claims 1 to 11, wherein the fetuin polypeptide is used in a method for diagnosis of calcification in bones, in particular when the bone metabolism is pathologic.

13. A diagnostic kit comprising the fetuin polypeptide as defined in any of claims 2 to 12 and optionally further comprising (i) one or more pharmaceutically acceptable carriers and/or (ii) a user manual.


SEQUENCE LISTING

<110>  Rheinisch-Westfälische Technische Hochschule Aachen

<120>  In Vivo Imaging of Calcified Lesions

<130>  P67137EP

<160>  4

<170>  PatentIn version 3.5

<210>  1
<211>  367
<212>  PRT
<213>  Homo sapiens

<400>  1

Met Lys Ser Leu Val Leu Leu Leu Cys Leu Ala Gln Leu Trp Gly Cys
1               5                   10                  15

His Ser Ala Pro His Gly Pro Gly Leu Ile Tyr Arg Gln Pro Asn Cys
            20                  25                  30

Asp Asp Pro Glu Thr Glu Glu Ala Ala Leu Val Ala Ile Asp Tyr Ile
        35                  40                  45

Asn Gln Asn Leu Pro Trp Gly Tyr Lys His Thr Leu Asn Gln Ile Asp
    50                  55                  60

Glu Val Lys Val Trp Pro Gln Gln Pro Ser Gly Glu Leu Phe Glu Ile
65                  70                  75                  80

Glu Ile Asp Thr Leu Glu Thr Thr Cys His Val Leu Asp Pro Thr Pro
                85                  90                  95

Val Ala Arg Cys Ser Val Arg Gln Leu Lys Glu His Ala Val Glu Gly
            100                 105                 110

Asp Cys Asp Phe Gln Leu Leu Lys Leu Asp Gly Lys Phe Ser Val Val
        115                 120                 125

Tyr Ala Lys Cys Asp Ser Ser Pro Asp Ser Ala Glu Asp Val Arg Lys
    130                 135                 140

Val Cys Gln Asp Cys Pro Leu Leu Ala Pro Leu Asn Asp Thr Arg Val
145                 150                 155                 160

Val His Ala Ala Lys Ala Ala Leu Ala Ala Phe Asn Ala Gln Asn Asn
                165                 170                 175

Gly Ser Asn Phe Gln Leu Glu Glu Ile Ser Arg Ala Gln Leu Val Pro
            180                 185                 190

Leu Pro Pro Ser Thr Tyr Val Glu Phe Thr Val Ser Gly Thr Asp Cys
        195                 200                 205

Val Ala Lys Glu Ala Thr Glu Ala Ala Lys Cys Asn Leu Leu Ala Glu
    210                 215                 220

Lys Gln Tyr Gly Phe Cys Lys Ala Thr Leu Ser Glu Lys Leu Gly Gly
225                 230                 235                 240

Ala Glu Val Ala Val Thr Cys Met Val Phe Gln Thr Gln Pro Val Ser
                245                 250                 255

Ser Gln Pro Gln Pro Glu Gly Ala Asn Glu Ala Val Pro Thr Pro Val
            260                 265                 270

Val Asp Pro Asp Ala Pro Pro Ser Pro Pro Leu Gly Ala Pro Gly Leu
        275                 280                 285

Pro Pro Ala Gly Ser Pro Pro Asp Ser His Val Leu Leu Ala Ala Pro
    290                 295                 300

Pro Gly His Gln Leu His Arg Ala His Tyr Asp Leu Arg His Thr Phe
305                 310                 315                 320

Met Gly Val Val Ser Leu Gly Ser Pro Ser Gly Glu Val Ser His Pro
                325                 330                 335

Arg Lys Thr Arg Thr Val Val Gln Pro Ser Val Gly Ala Ala Ala Gly
            340                 345                 350

Pro Val Val Pro Pro Cys Pro Gly Arg Ile Arg His Phe Lys Val
        355                 360                 365

<210>  2
<211>  345
<212>  PRT
<213>  Mus musculus

<400>  2

Met Lys Ser Leu Val Leu Leu Leu Cys Phe Ala Gln Leu Trp Gly Cys
1               5                   10                  15

Gln Ser Ala Pro Gln Gly Thr Gly Leu Gly Phe Arg Glu Leu Ala Cys
            20                  25                  30

Asp Asp Pro Glu Ala Glu Gln Val Ala Leu Leu Ala Val Asp Tyr Leu
        35                  40                  45

Asn Asn His Leu Leu Gln Gly Phe Lys Gln Val Leu Asn Gln Ile Asp
    50                  55                  60

Lys Val Lys Val Trp Ser Arg Arg Pro Phe Gly Val Val Tyr Glu Met
65                  70                  75                  80

Glu Val Asp Thr Leu Glu Thr Thr Cys His Ala Leu Asp Pro Thr Pro
                85                  90                  95

Leu Ala Asn Cys Ser Val Arg Gln Leu Thr Glu His Ala Val Glu Gly
            100                 105                 110

Asp Cys Asp Phe His Ile Leu Lys Gln Asp Gly Gln Phe Arg Val Met
        115                 120                 125

His Thr Gln Cys His Ser Thr Pro Asp Ser Ala Glu Asp Val Arg Lys
    130                 135                 140

Leu Cys Pro Arg Cys Pro Leu Leu Thr Pro Phe Asn Asp Thr Asn Val
145                 150                 155                 160

Val His Thr Val Asn Thr Ala Leu Ala Ala Phe Asn Thr Gln Asn Asn
                165                 170                 175

Gly Thr Tyr Phe Lys Leu Val Glu Ile Ser Arg Ala Gln Asn Val Pro
            180                 185                 190

Leu Pro Val Ser Thr Leu Val Glu Phe Val Ile Ala Ala Thr Asp Cys
        195                 200                 205

Thr Ala Lys Glu Val Thr Asp Pro Ala Lys Cys Asn Leu Leu Ala Glu
    210                 215                 220

Lys Gln His Gly Phe Cys Lys Ala Asn Leu Met His Asn Leu Gly Gly
225                 230                 235                 240

Glu Glu Val Ser Val Ala Cys Lys Leu Phe Gln Thr Gln Pro Gln Pro
                245                 250                 255

Ala Asn Ala Asn Ala Val Gly Pro Val Pro Thr Ala Asn Ala Ala Leu
            260                 265                 270

Pro Ala Asp Pro Pro Ala Ser Val Val Val Gly Pro Val Val Val Pro
        275                 280                 285

Arg Gly Leu Ser Asp His Arg Thr Tyr His Asp Leu Arg His Ala Phe
    290                 295                 300

Ser Pro Val Ala Ser Val Glu Ser Ala Ser Gly Glu Thr Leu His Ser
305                 310                 315                 320

Pro Lys Val Gly Gln Pro Gly Ala Ala Gly Pro Val Ser Pro Met Cys
                325                 330                 335

Pro Gly Arg Ile Arg His Phe Lys Ile
            340                 345

<210>  3

<211>  359
<212>  PRT
<213>  Bos taurus

<400>  3

Met Lys Ser Phe Val Leu Leu Phe Cys Leu Ala Gln Leu Trp Gly Cys
1               5                   10                  15

His Ser Ile Pro Leu Asp Pro Val Ala Gly Tyr Lys Glu Pro Ala Cys
            20                  25                  30

Asp Asp Pro Asp Thr Glu Gln Ala Ala Leu Ala Ala Val Asp Tyr Ile
        35                  40                  45

Asn Lys His Leu Pro Arg Gly Tyr Lys His Thr Leu Asn Gln Ile Asp
    50                  55                  60

Ser Val Lys Val Trp Pro Arg Arg Pro Thr Gly Glu Val Tyr Asp Ile
65                  70                  75                  80

Glu Ile Asp Thr Leu Glu Thr Thr Cys His Val Leu Asp Pro Thr Pro
                85                  90                  95

Leu Ala Asn Cys Ser Val Arg Gln Gln Thr Gln His Ala Val Glu Gly
            100                 105                 110

Asp Cys Asp Ile His Val Leu Lys Gln Asp Gly Gln Phe Ser Val Leu
        115                 120                 125

Phe Thr Lys Cys Asp Ser Ser Pro Asp Ser Ala Glu Asp Val Arg Lys
    130                 135                 140

Leu Cys Pro Asp Cys Pro Leu Leu Ala Pro Leu Asn Asp Ser Arg Val
145                 150                 155                 160

Val His Ala Val Glu Val Ala Leu Ala Thr Phe Asn Ala Glu Ser Asn
                165                 170                 175

Gly Ser Tyr Leu Gln Leu Val Glu Ile Ser Arg Ala Gln Phe Val Pro
            180                 185                 190

Leu Pro Val Ser Val Ser Val Glu Phe Ala Val Ala Ala Thr Asp Cys
        195                 200                 205

Ile Ala Lys Glu Val Val Asp Pro Thr Lys Cys Asn Leu Leu Ala Glu
    210                 215                 220

Lys Gln Tyr Gly Phe Cys Lys Gly Ser Val Ile Gln Lys Ala Leu Gly
225                 230                 235                 240

Gly Glu Asp Val Arg Val Thr Cys Thr Leu Phe Gln Thr Gln Pro Val
                245                 250                 255

Ile Pro Gln Pro Gln Pro Asp Gly Ala Glu Ala Glu Ala Pro Ser Ala
            260                 265                 270

Val Pro Asp Ala Ala Gly Pro Thr Pro Ser Ala Ala Gly Pro Pro Val
        275                 280                 285

Ala Ser Val Val Val Gly Pro Ser Val Val Ala Val Pro Leu Pro Leu
    290                 295                 300

His Arg Ala His Tyr Asp Leu Arg His Thr Phe Ser Gly Val Ala Ser
305                 310                 315                 320

Val Glu Ser Ser Ser Gly Glu Ala Phe His Val Gly Lys Thr Pro Ile
                325                 330                 335

Val Gly Gln Pro Ser Ile Pro Gly Gly Pro Val Arg Leu Cys Pro Gly
            340                 345                 350

Arg Ile Arg Tyr Phe Lys Ile
        355

<210>  4
<211>  10
<212>  PRT
<213>  Homo sapiens

<400>  4

Glu Gly Asp Cys Asp Phe Gln Leu Leu Lys
1               5                   10